(19) Europäisches Patentamt

European Patent Office

Office européen des brevets

(11) Veröffentlichungsnummer: **0 198 259 B1**

(12) **EUROPÄISCHE PATENTSCHRIFT**

(45) Veröffentlichungstag der Patentschrift: **10.06.92**

(21) Anmeldenummer: **86103752.1**

(22) Anmeldetag: **19.03.86**

(51) Int. Cl.⁵: **A61K 37/02**, C07K 3/28, G01N 33/68, A61K 39/395

(54) Verfahren zur immunologischen Bestimmung von Heparansulfat-Proteoglykan, Verfahren zur Herstellung bzw. Reinigung von dafür geeignetem Heparansulfat-Proteoglykan aus basalmembranhaltigen Geweben.

(30) Priorität: **28.03.85 DE 3511199**

(43) Veröffentlichungstag der Anmeldung:
**22.10.86 Patentblatt 86/43**

(45) Bekanntmachung des Hinweises auf die Patenterteilung:
**10.06.92 Patentblatt 92/24**

(84) Benannte Vertragsstaaten:
**AT BE CH DE FR GB IT LI LU NL SE**

(56) Entgegenhaltungen:
**EP-A- 21 152**

**THE JOURNAL OF BIOLOGICAL CHEMISTRY, Band 258, Nr. 3, 10. Februar 1983, (US); A.OOHIRA et al., Seiten 2014-2021**

**PROCEEDINGS OF THE NATIONAL ACADEMY OF SCIENCE U.S.A., Band 82, Mai 1985; J.L.STOW et al., Seiten 3296-3300**

(73) Patentinhaber: **Max-Planck-Gesellschaft zur Förderung der Wissenschaften e.V.**
**Bunsenstrasse 10**
**W-3400 Göttingen(DE)**

Patentinhaber: **HOECHST AKTIENGESELLSCHAFT**
**Postfach 80 03 20**
**W-6230 Frankfurt am Main 80(DE)**

(72) Erfinder: **Timpl, Rupert, Dr.**
**Julius Haerlinstr. 3**
**W-8035 Gauting(DE)**
Erfinder: **Paulsson, Mats, Dr.**
**Röntgenstr. 1a**
**W-8033 Martinsried(DE)**
Erfinder: **Brocks, Dietrich, Dr.**
**Goethering 9**
**W-6200 Wiesbaden 42(DE)**

(74) Vertreter: **Klein, Otto, Dr. et al**
**Hoechst AG Zentrale Patentabteilung Postfach 80 03 20**
**W-6230 Frankfurt am Main 80(DE)**

EP 0 198 259 B1

CHEMICAL ABSTRACTS, Band 103, Nr. 21, 25 November 1985, Columbus, OH (US); D.C.SELDIN et al., Seite 301, Nr. 174245u

LABORATORY INVESTIGATION, Band 48, Nr. 3, 1983, The United States Canadian Division of the International Academy of Pathology, US; L.A.MYNDERSE etal.

CHEMICAL ABSTRACTS, Band 101, Nr. 15, 08 Oktober 1984, Columbus, OH (US); S.FUJIWARA et al., Seiten 267-268, Nr. 125281c

PROCEEDINGS OF THE NATL. ACADEMY OF SCIENCES USA, Band 77, 1980; Seiten 4494-4498

JOURNAL MOLECULAR BIOLOGY, Band 197, 1987; Seiten 297-313

EUR. JOURNAL BIOCHEMISTRY, Band 143, 1984; Seiten 145-157

EMBO J., Band 4, Nr. 4, 1985; Seiten 905-912

## Beschreibung

Heparansulfat-Proteoglykan kommt in Basalmembranen vor und wirkt dort als ionenselektiver Filter. In pathologischen Situationen wie z. B. Diabetes mellitus wurde eine Verringerung dieses Proteoglykans in Basalmembranen beschrieben. Diese Verringerung wird in ursächlichem Zusammenhang mit dem Auftreten diabetischer Spätkomplikationen wie Nephropathie gesehen. Die für den Nachweis der Verringerung verwendeten Verfahren setzen eine Gewebsextraktion und damit die Verfügbarkeit von Gewebe voraus. Es wäre wünschenswert, eine solche Veränderung durch eine Serumanalyse bestimmen zu können.

Überraschenderweise wurde nun gefunden, daß mit Hilfe des erfindungsgemäßen Verfahrens Heparansulfat-Proteoglykan niederer Dichte aus basalmembranhaltigem Gewebe rein isoliert werden kann. Unter Verwendung dieses Antigens und der entsprechenden hochspezifischen Antikörper kann eine hochsensitive immunologische Bestimmungsmethode erstellt werden. Mit dieser erfindungsgemäßen Bestimmungsmethode ist der Nachweis auch von geringen Mengen (ng/ml) Heparansulfat-Proteoglykan niederer Dichte in Körperflüssigkeiten, insbesondere im Blut bzw. im Serum möglich.

Dies bedeutet eine wertvolle Erweiterung der Diagnostikmethoden. Üblicherweise werden im Serum normaler Individuen Konzentrationen von Heparansulfat-Proteoglykan niederer Dichte im Bereich von 100-150 ng/ml gefunden. Diese Konzentration ist bei Basalmembranveränderungen, wie z. B. bei experimentell erzeugtem Diabetes mellitus, statistisch signifikant erhöht. Mit Hilfe des erfindungsgemäßen Verfahrens sind solche Veränderungen nunmehr schnell und sicher erfaßbar.

Die Erfindung betrifft:

1. Ein verfahren zur Herstellung von Heparansulfat-Proteoglykan niederer Dichte ($\rho$ = 1,33-1,39), das dadurch gekennzeichnet ist, daß Basalmembrangewebe aus Humanplacenta

a) mit 3,4 bis 4 molarer Alkalisalzlösung und/oder mit 0,1 bis 1 molarer Alkalisalzlösung in Gegenwart von Proteaseinhibitoren homopenisiert und extrahiert wird,

b) der Rückstand mit einer 1 bis 8 molaren chaotropen Salzlösung in Gegenwart von Proteaseinhibitoren extrahiert wird und aus dem erhaltenen Extrakt

c) das genannte Heparansulfat-Proteoglykan niederer Dichte durch Ionenaustauschchromatographie mit anschließender Molekularsiebchromatographie und Auftrennung im Dichtegradienten gereinigt wird.

2. Ein Heparansulfat-Proteoglykan niederer Dichte ($\rho$ = 1,33-1,39), das nach dem unter 1. beschriebenen Verfahren erhältlich ist.

3. Ein verfahren zur Herstellung hochspezifischer Seren mit Antikörpern gegen Heparansulfat-Proteoglykan niederer Dichte ($\rho$ = 1,33-1,39) erhältlich nach dem unter 1. beschriebenen Verfahren, das dadurch gekennzeichnet ist, daß Versuchstiere mit genanntem Heparansulfat-Proteoglykan niederer Dichte immunisiert werden und ihr Serum gewonnen wird.

4. Ein hochspezifisches Serum mit Antikörpern gegen Heparansulfat-Proteoglykan niederer Dichte ($\rho$ = 1,33-1,39), welches nach dem unter 3. beschriebenen Verfahren erhältlich ist.

5. Ein verfahren zur immunologischen Bestimmung von Heparansulfat-Proteoglykan niederer Dichte, das dadurch gekennzeichnet ist, daß das Antigen Heparansulfat-Proteoglykan niederer Dichte und/oder dessen antigene Determinaten in Körperflüssigkeiten mit Hilfe von Antikörpern, erhältlich nach dem unter 3. beschriebenen Verfahren, bestimmt werden.

In der folgenden Beschreibung werden die aufgeführten Punkte näher erläutert bzw. in den Ansprüchen definiert.

Mit dem erfindungsgemäßen verfahren läßt sich Heparansulfat-Proteoglykan niederer Dichte aus Humanplacenta gewinnen.

Bei diesem Verfahren wird auf die Reindarstellung von Basalmembranen völlig verzichtet. Die Isolierung des Antigens erfolgt mit Hilfe hoher Konzentrationen chaotroper Salzlösungen nach der Entfernung störender Proteine, die mit Alkalisalzen extrahiert werden können.

Geeignete chaotrope Salze sind beispielsweise Kaliumrhodanid, Guanidinrhodanid und Guanidinchlorid. Vorzugsweise wird Guanidinchlorid verwendet. Diese Salze werden in Konzentrationen von 1 - 8 molar eingesetzt wobei der Bereich von 4 bis 7 molar bevorzugt ist.

Zur Entfernung störender Proteine gibt es, je nach Verunreinigung, mehrere Möglichkeiten:

1. Das Gewebe wird in wäßriger Pufferlösung mit so hohen Alkalisalzkonzentrationen, daß keine Gewebsbestandteile in Lösung gehen, zerkleinert und homogenisiert. Bevorzugt werden NaCL oder KCl verwendet in 3,4 - 4 molarer Konzentration.

2. Man arbeitet in Gegenwart niedriger Salzkonzentrationen, bevorzugt Pufferlösungen mit 0,1 - 1 molarer Konzentration an NaCL oder KCl.

3. Man kombiniert 1 und 2.

Heparansulfat-Proteoglykan niederer Dichte wird nach der Vorextraktion wie oben erwähnt isoliert. Sowohl bei der eigentlichen Extraktion des Antigens wie auch bei der Vorreinigung wird vorzugsweise in Gegenwart von Proteaseinhibitoren und bei neutralem pH gearbeitet.

Aus dem Extrakt wird dann Heparansulfat-Proteoglykan niederer Dichte durch Ionenaustauschchromatographie, vorzugsweise an einem schwach basischen Kationenaustauscher wie z. B. DEAE Cellulose oder ein DEAE-vernetztes Dextran (z.B. [R] Sephadex, Pharmacia Fine Chemicals Inc.) in Gegenwart hoher Konzentration von Harnstoff bzw. dessen Derivaten gereinigt.

Eine erste Feinreinigung erfolgt über Molekularsiebchromatographie. Man erhält so ein Produkt von ausreichender Reinheit zur Herstellung von Antikörpern.

Eine anschließende Dichtegradientenzentrifugation liefert ein Heparansulfat-Proteoglykan niederer Dichte ($\rho$ = 1,33-1,39 g/ml), das protein- und nucleinsäurefrei ist.

Für das erfindungsgemäße Bestimmungsverfahren ist es entscheidend, daß ein geeignetes Antiserum gegen Heparansulfat-Proteoglykan niederer Dichte zur Verfügung steht. Die Herstellung der Antiseren kann in üblicher Weise durch subcutane oder intramusculäre Injektion an Versuchstieren, wie Meerschweinchen, Ziegen, Schafe, Esel und vorzugsweise Kaninchen, erfolgen. Zweckmäßig wird dabei in Gegenwart von komplettem Freund'schen Adjuvans gearbeitet. Es können die in diesen Fällen üblichen Antigendosen angewendet werden. Die bevorzugte Dosis am Kaninchen ist 0,5 bis 1 mg pro Tier. Das gebildete Antiserum wird dann in der dem Fachmann bekannten Weise gewonnen und kann als solches verwendet werden. Es ist auch möglich, die im Serum vorhandenen Antikörper vor der weiteren Verwendung noch zu reinigen. Eine bevorzugte Methode dabei ist die Affinitätschromatographie, beispielsweise an Affinitätsmatrices, an die das Antigen kovalent gebunden wurde, z.B. bromcyanaktivierte ®Sepharose.

Die, für die im folgenden aufgeführte immunologische Bestimmung, notwendige Markierung des Antigens kann in den für Proteinmarkierung bekannten Methoden erfolgen. Es wird bevorzugt radioaktiv-, enzym- oder fluoreszenzmarkiert. Im Falle der radioaktiven Markierung wird als Radionuklid bevorzugt Jod 125 verwendet. Die Markierung kann dann nach der bekannten Chloramin T Methode (Int. Arch. Allergy 29, 185, 1966) erfolgen.

Das nach den obengenannten erfindungsgemäßen Verfahren isolierte Heparansulfat-Proteoglykan niederer Dichte sowie das Antiserum ermöglichen, wie bereits erwähnt, die erfindungsgemäße Bestimmung von Heparansulfat-Proteoglykan niederer Dichte in Körperflüssigkeiten sowie dessen antigene Determinanten, insbesondere im Serum, unter Anwendung der an sich bekannten immunologischen Nachweismethoden.

Es können bei diesen Verfahren sowohl die bekannten Radio-Immun-Assay-(RIA)-Varianten, als auch die Enzym-Immun-Assay-Varianten und analoge Bestimmungen unter Verwendung andersartiger Markierung, beispielsweise Fluoreszenzmarkierung, Farbstoffmarkierung und dergleichen angewendet werden. Derartige Methoden sind dem Fachmann bekannt und sollen hier nicht im einzelnen aufgeführt werden.

Bei diesen Nachweisreaktionen konkurriert das markierte Heparansulfat-Proteoglykan niederer Dichte in bekannter Weise mit unmarkiertem Antigen, das in der zu bestimmenden Probe vorhanden ist, um den Antikörper, so daß im gebildeten Antigen-Antikörper-Komplex die Menge markierten Antigens umso geringer ist, je mehr nicht markierte Antigene in der zu bestimmenden Probe enthalten sind. Es kann entweder die Markierung des Komplexes, beispielsweise die Radioaktivität oder die Enzymaktivität, oder die Markierung des Überstandes nach Abtrennung des Antigen-Antikörper-Komplexes verwendet werden, um anhand einer Eichkurve, die mittels Proben bekannten Gehaltes an Heparansulfat-Proteoglykan niederer Dichte erstellt wurde, die in der zu untersuchenden Probe enthaltenen Mengen Antigen festzustellen. Es wird bevorzugt die Markierung im Komplex bestimmt.

Eine bevorzugte Ausführungsform der erfindungsgemäßen immunologischen Methode besteht darin, die Abtrennung des mit dem spezifischen Antiserum gebildeten Antigen-Antikörper-Komplexes mittels eines zweiten Antikörpers durchzuführen. Bevorzugt wird dazu ein gegen Immunglobulin G, der für die Gewinnung des spezifischen Antiserums verwendeten Tierart gerichteter Antikörper verwendet. Die Abtrennung des Antigen-Antikörper-Komplexes von der Lösung kann mit hierfür üblichen Methoden wie Fällung, Zentrifugation oder Abfiltrieren erfolgen.

Alternativ wird das Antiserum bzw. der zweite Antikörper an einen festen Träger gebunden. Nach Abtrennung des Antigen-Antikörper-Komplexes wird dann die im Komplex enthaltene, bzw. die in der Lösung verbliebene Radioaktivität oder andere Markierung bestimmt.

Eine andere Variante zur Bestimmung von Heparansulfat-Proteoglykan niederer Dichte kann auch dergestalt durchgeführt werden, daß der Antikörper gegen Immunglobulin G der betreffenden Tierart, z. B. vom Kaninchen, markiert ist, wobei ebenfalls die schon aufgeführten Markierungen geeignet sind, bevorzugt jedoch die Enzymmarkierung verwendet wird. Bei einem Enzym-Immun-Assay z.B. wird der nach Bildung des Antigen-Antikörper-Komplexes verbleibende Teil des Anti-Heparansulfat-Proteoglykan(niederer Dichte)-Serums bestimmt, indem man diesen an trägergebundenes Heparansulfat-Proteoglykan niederer Dichte bindet und anschließend mit enzymmarkiertem Antikörper gegen Immunglobulin G des Kaninchens zur Reaktion bringt. Die Menge gebundenen enzymmarkierten Antikörpers wird anschließend durch Messung der Enzymreaktion bestimmt und ist der unbekannten Menge von Heparansulfat-Proteoglykan niederer Dichte in der Probe indirekt proportional.

Die folgenden Beispiele erläutern die Erfindung weiter.

Beispiel 1

Herstellung von Heparansulfat-Proteoglykan niederer Dichte

Als Ausgangsmaterial wird Humanplacenta verwendet. Das Gewebe wird zwei- bis dreimal in einem 20-fachen Überschuß an 0,15 M NaCl, 0,05 M Tris/HCl (pH 7,4) in Gegenwart der Proteaseinhibitoren Phenylmethansulfonsäurefluorid (PMSF) (2,5 mM), Quecksilbersalz der p-Chlorbenzoesäure (3 mg/ml), EDTA (10 mM) und N-Ethylmaleimid (NEM) (2,5 mM) homogenisiert und extrahierbares Protein durch Zentrifugation entfernt.

Der Rückstand wird dann zweimal mit 6 M Guanidinchlorid, 0,05 M Tris/HCl pH 7,4 in Gegenwart der Proteaseinhibitoren bei 4°C extrahiert. Der Extrakt enthält Heparansulfat-Proteoglykan. Durch Ionenaustauschchromatographie an DEAE Cellulose die in 7 M Harnstoff, 0,05 M Tris/HCl (pH 8,6), 0,5 mM EDTA, 0,5 mM NEM, 0,5 mM PMSF bei 4°C äquilibriert wurde und Elution mit einem Gradienten von 0-0,6 M NaCl wird Heparansulfat-Proteoglykan, welches in der zweiten Hälfte des Gradienten eluiert, weiter gereinigt.

Nach Molekularsiebchromatographie über ein Allyldextran vernetzt mit N-N'-Methylenbisacrylamid (z.B. (R) Sephacryl S400, Pharmacia Fine Chemicals Inc.) äquilibriert in 6 M Guanidinchlorid 0,05 M Tris/HCl pH 7,4 mit den bei der DEAE Chromatographie verwendeten Inhibitoren und CsCl-Dichtegradientenzentrifugation in 6 M Guanidinchlorid-Puffer mit einer anfänglichen Dichte von 1,34 g/ml (> 100.000 g, 8 h, 18°C) erhält man in der zentralen Portion des Gradienten ($\rho$ = 1,33-1,39 g/ml) ein Heparansulfat-Proteoglykan niederer Dichte, das frei ist von Heparansulfat-Proteoglykan hoher Dichte, Glykoproteinen und Nukleinsäuren.

Beispiel 2

Herstellung des markierten Antigens

25 ng von Heparansulfat-Proteoglykan niederer Dichte werden mit 0,5 mCi Jod 125 nach der Chloramin T-Methode markiert und ungebundenes Jod durch Dialyse oder Gelfiltration an einem Polyacrylamid-Gel (z.B. (R) Biogel P2, Pharmacia Fine Chemicals Inc.) entfernt.

Beispiel 3

Durchführung der immunologischen Bestimmung (RIA)

Alle für die immunologische Bestimmung notwendigen Schritte werden in Gegenwart von 0,04% eines nichtionischen Detergens wie z.B. Tween 20, ein polyethoxyliertes Sorbit-Monolaurats, durchgeführt. Bindungskurven werden mit 1 ng markiertem Heparansulfat-Proteoglykan bestimmt.

Die Konzentration an Heparansulfat-Proteoglykan in einer unbekannten Probe von Serum oder anderen Körperflüssigkeiten wird in folgendem Inhibitionstest bestimmt:

Eine bestimmte Menge des spezifischen Antikörpers oder Antiserums wird mit der unbekannten Probe 16 h bei 4°C vorinkubiert und nach Zugabe von 1 ng markierten Antigens weitere 8 Stunden bei 4°C inkubiert. Danach wird ein Überschuß von Antikörpern gegen Kaninchen-Immunoglobulin G zugesetzt und nach weiteren 16 h bei 4°C das im Immunkomplex gebundene Antigen durch Zentrifugation abgetrennt. Die Inhibitonsaktivität der unbekannten Probe wird mit der Aktivität einer Standard-Konzentration nicht-markierten Antigens verglichen.

Beispiel 4

Herstellung des Antiserums

Kaninchen werden mit 0,5 - 1 mg Heparansulfat-Proteoglykan niederer Dichte pro Tier immunisiert, indem die Antigenlösung, gemischt mit gleichen Volumen komplettem Freund'schen Adjuvans, subcutan oder intramuskulär injiziert wird. 4 Wochen nach der ersten Injektion wird eine gleiche Menge Antigenlösung, gemischt mit komplettem Freund'schen Adjuvans subcutan oder intramuskulär injiziert. 4 bis 8 Wochen nach der 2. Injektion wird das Blut gewonnen und daraus nach Gerinnung das Antiserum erhalten.

**Patentansprüche**

**Patentansprüche für folgende Vertragsstaaten : BE, CH, DE, FR, GB, IT, LI, LU, NL, SE**

1. Verfahren zur Herstellung von Heparansulfat-Proteoglykan niederer Dichte ($\rho$ = 1,33-1,39), dadurch gekennzeichnet, daß Basalmembrangewebe aus Humanplacenta

   a) mit 3,4 bis 4 molarer Alkalisalzlösung und/oder mit 0,1 bis 1 molarer Alkalisalzlösung in Gegenwart von Proteaseinhibitoren homogenisiert und extrahiert wird,

   b) der Rückstand mit einer 1 bis 8 molaren chaotropen Salzlösung in Gegenwart von Proteaseinhibitoren extrahiert wird und aus dem erhaltenen Extrakt
c) das genannte Heparansulfat-Proteoglykan niederer Dichte durch Ionenaustauschchromatographie mit anschließender Molekularsiebchromatographie und Auftrennung im Dichtegradienten gereinigt wird.

2. Verfahren zur Herstellung hochspezifischer Seren mit Antikörpern gegen Heparansulfat-Proteoglykan niederer Dichte ($\rho$ = 1,33-1,39) erhältlich nach Anspruch 1, dadurch gekennzeichnet, daß Versuchstiere mit genanntem Heparansulfat-Proteoglykan niederer Dichte immunisiert werden und ihr Serum gewonnen wird.

3. Verfahren zur immunologischen Bestimmung von Heparansulfat-Proteoglykan niederer Dichte, dadurch gekennzeichnet, daß das Antigen Heparansulfat-Proteoglykan niederer Dichte und/oder dessen antigene Determinanten in Körperflüssigkeiten mit Hilfe von Antikörpern erhältlich nach Anspruch 2 bestimmt werden.

4. Heparansulfat-Proteoglykan niederer Dichte ($\rho$ = 1,33-1,39), das nach dem Verfahren in Anspruch 1 erhältlich ist.

5. Hochspezifisches Serum mit Antikörpern gegen Heparansulfat-Proteoglykan niederer Dichte ($\rho$ = 1,33-1,39), welches nach dem Verfahren in Anspruch 2 erhältlich ist.

6. Verfahren nach Anspruch 3, dadurch gekennzeichnet, daß
a) das genannte Antigen oder dessen Antigene Determinaten in Körperflüssigkeiten mit einer bestimmten, in bezug auf die zu untersuchende Probe überschüssigen Menge von Antikörpern zur Reaktion gebracht wird, wobei ein Antigen-Antikörperkomplex gebildet wird, und
b) die genannten Antikörper aus dem genannten Antigen-Antikörper quantifiziert werden.

7. Verfahren nach Anspruch 6, dadurch gekennzeichnet, daß zur Quantifizierung
a) die in bezug auf die Probe überschüssige Menge an Antikörpern mit einem Überschuß markierter Antigene, zusätzlich zu den in der zu untersuchenden Probe befindlichen Antigenen, zur Reaktion gebracht werden, und
b) das entstandene Produkt in Antigen-Antikörper-Komplex und Überstand aufgetrennt wird, und
c) die Menge des markierten Antigens entweder im Komplex oder im Überstand nach Abtrennung des Komplexes bestimmt wird.

8. Verfahren nach Anspruch 3, dadurch gekennzeichnet, daß das markierte Antigen radioaktiv, enzym- oder fluoreszenzmarkiert ist.

9. Verfahren nach Anspruch 6, dadurch gekennzeichnet, daß zur Quantifizierung
a) die in bezug auf die Probe überschüssige Menge an Antikörper mit einem Überschuß an Antigen, das an einen Träger gebunden ist, zur Reaktion gebracht wird, und dann
b) den Antigen-Antikörper-Komplex aus a) mit einem Überschuß eines markierten zweiten Antikörpers zur Reaktion bringt, und
c) das aus b) entstandene Produkt in Antigen-Antikörper-Komplex und Überstand mit freien markierten zweiten Antikörpern auftrennt und
d) die Menge des markierten zweiten Antikörpers entweder im Komplex oder im Überstand bestimmt.

10. Verfahren nach Anspruch 8, dadurch gekennzeichnet, daß der markierte zweite Antikörper radioaktiv, enzym- oder fluoreszenzmarkiert ist.

**Patentansprüche für folgenden Vertragsstaat : AT**

1. Verfahren zur Herstellung von Heparansulfat-Proteoglykan niederer Dichte ($\rho$ = 1,33-1,39), dadurch gekennzeichnet, daß Basalmembrangewebe aus Humanplacenta
a) mit 3,4 bis 4 molarer Alkalisalzlösung und/oder mit 0,1 bis 1 molarer Alkalisalzlösung in Gegenwart von Proteaseinhibitoren extrahiert wird,
b) der Rückstand mit einer 1 bis 8 molaren chaotropen Salzlösung in Gegenwart von Proteaseinhibitoren extrahiert wird und aus dem erhaltenen Extrakt
c) das genannte Heparansulfat-Proteoglykan niederer Dichte durch Ionenaustauschchromatographie mit anschließender Molekularsiebchromatographie und Auftrennung im Dichtegradienten gereinigt wird.

2. Verfahren zur Herstellung hochspezifischer Seren mit Antikörpern gegen Heparansulfat-Proteoglykan niederer Dichte ($\rho$ = 1,33-1,39) erhältlich nach Anspruch 1, dadurch gekennzeichnet, daß Versuchstiere mit genanntem Heparansulfat-Proteoglykan niederer Dichte immunisiert werden und ihr Serum gewonnen wird.

3. Verfahren zur immunologischen Bestimmung von Heparansulfat-Proteoglykan niederer Dichte, dadurch gekennzeichnet, daß das Antigen Heparansulfat-Proteoglykan niederer Dichte und/oder dessen antigene Determinanten in Körperflüssigkeiten mit Hilfe von Antikörpern erhältlich nach Anspruch bestimmt werden.

4. Verfahren nach Anspruch 3, dadurch gekennzeichnet, daß
a) das genannte Antigen oder dessen antigene Determinanten in Körperflüssigkeiten mit einer bestimmten, in bezug auf die zu untersuchende Probe überschüssigen Menge von Antikörpern zur Reaktion gebracht wird, wobei ein Antigen-Antikörper-Komplex gebildet wird, und
b) die genannten Antikörper aus dem genannten Antigen-Antikörper-Komplex quantifiziert werden.

5. Verfahren nach Anspruch 4, dadurch gekennzeichnet, daß zur Quantifizierung
a) die in bezug auf die Probe überschüssige Menge an Antikörpern mit einem Überschuß markierter Antigene, zusätzlich zu den in der zu untersuchenden Probe befindlichen Antigenen, zur Reaktion gebracht werden, und
b) das entstandene Produkt in Antigen-Antikörper-Komplex und Überstand aufgetrennt wird, und
c) die Menge des markierten Antigens entweder im Komplex oder im Überstand nach Abtrennung des Komplexes bestimmt wird.

6. Verfahren nach Anspruch 5, dadurch gekennzeichnet, daß das markierte Antigen radioaktiv, enzym- oder fluoreszenzmarkiert ist.

7. Verfahren nach Anspruch 4, dadurch gekennzeichnet, daß zur Quantifizierung
a) die in bezug auf die Probe überschüssige Menge an Antikörper mit einem Überschuß an Antigen, das an einen Träger gebunden ist, zur Reaktion gebracht wird, und dann
b) den Antigen-Antikörper-Komplex aus a) mit einem Überschuß eines markierten zweiten Antikörpers zur Reaktion bringt, und
c) das aus b) entstandene Produkt in Antigen-Antikörper-Komplex und Überstand mit freien markierten zweiten Antikörpern auftrennt und
d) die Menge des markierten zweiten Antikörpers entweder im Komplex oder im Überstand bestimmt.

8. Verfahren nach Anspruch 8, dadurch gekennzeichnet, daß der markierte zweite Antikörper radioaktiv, enzym- oder fluoreszenzmarkiert ist.

## Claims

**Claims for the following Contracting States :
BE, CH, DE, FR, GB, IT, LI, LU, NL, SE**

1. A process for the preparation of low density ($\rho$ = 1.33-1.39) heparan sulfate-proteoglycan, which comprises basement membrane tissue from human placenta
a) being homogenized and extracted with 3.4 to 4 molar alkali metal salt solution and/or with 0.1 to 1 molar alkali metal salt solution in the presence of protease inhibitors,
b) the residue being extracted with a 1 to 8 molar chaotropic salt solution in the presence of protease inhibitors and, from the resulting extract,
c) the said low-density heparan sulfate-proteoglycan being purified by ion exchange chromatography followed by molecular sieve chromatography and fractionation in a density gradient.

2. A process for the production of highly specific sera containing antibodies against low-density ($\rho$ - 1.33-1.39) heparan sulfate-proteoglycan obtainable as claimed in claim 1, which comprises immunization of experimental animals with the said low-density heparan sulfate-proteoglycan, and obtaining of their serum.

3. A method for the immunological determination of low-density heparan sulfate-proteoglycan, which comprises determination of the antigen low-density heparan sulfateproteoglycan, and/or of its antigenic determinants, in body fluids, with the aid of antibodies obtainable as claimed in claim 2.

4. A low-density ($\rho$ = 1.33-1.39) heparan sulfateproteoglycan which is obtainable by the process as claimed in claim 1.

5. A highly specific serum containing antibodies against low-density ($\rho$ = 1.33-1.39) heparan sulfateproteoglycan, which is obtainable by the process as claimed in claim 2.

6. The method as claimed in claim 3, wherein
a) the said antigen or its antigenic determinants in body fluids is reacted with a defined amount, which is in excess relative to the sample which is to be investigated, of antibodies, there being formation of an antigen-antibody complex, and

b) the said antibodies from the said antigen-antibody complex are quantified.

7. The method as claimed in claim 6, wherein for the quantification
    a) the amount of antibodies, which is in excess relative to the sample, is reacted with an excess of labeled antigens, in addition to the antigens present in the sample to be investigated, and
    b) the resulting product is separated into antigenantibody complex and supernatant, and
    c) the amount of labeled antigen is determined either in the complex or in the supernatant after removal of the complex.

8. The method as claimed in claim 3, wherein the labeled antigen is radioactively, enzyme- or fluorescence-labeled.

9. The method as claimed in claim 6, wherein for the quantification
    a) the amount, which is in excess relative to the sample, of antibody, is reacted with an excess of antigen which is bound to a carrier, and then
    b) the antigen-antibody complex from a) is reacted with an excess of a second, labeled, antibody, and
    c) the product resulting from b) is separated into antigen-antibody complex and supernatant containing free, labeled, second antibodies, and
    d) the amount of the second, labeled, antibody is determined either in the complex or in the supernatant.

10. The method as claimed in claim 8, wherein the second, labeled, antibody is radioactively, enzyme- or fluorescence-labeled.

**Claims for the following Contracting State : AT**

1. A process for the preparation of low density ($\rho$ = 1.33-1.39) heparan sulfate-proteoglycan, which comprises basement membrane tissue from human placenta
    a) being extracted with 3.4 to 4 molar alkali metal salt solution and/or with 0.1 to 1 molar alkali metal salt solution in the presence of protease inhibitors,
    b) the residue being extracted with a 1 to 8 molar chaotropic salt solution in the presence of protease inhibitors and, from the resulting extract,
    c) the said low-density heparan sulfate-proteoglycan being purified by ion exchange chromatography followed by molecular sieve chromatography and fractionation in a density gradient.

2. A process for the production of highly specific sera containing antibodies against low-density ($\rho$ = 1.33-1.39) heparan sulfate-proteoglycan obtainable as claimed in claim 1, which comprises immunization of experimental animals with the said low-density heparan sulfate-proteoglycan, and obtaining of their serum.

3. A method for the immunological determination of low-density heparan sulfate-proteoglycan, which comprises determination of the antigen low-density heparan sulfateproteoglycan, and/or of its antigenic determinants, in body fluids, with the aid of antibodies obtainable as claimed in claim 2.

4. The method as claimed in claim 3, wherein
    a) the said antigen or its antigenic determinants in body fluids is reacted with a defined amount, which is in excess relative to the sample which is to be investigated, of antibodies, there being formation of an antigen-antibody complex, and
    b) the said antibodies from the said antigen-antibody complex are quantified.

5. The method as claimed in claim 4, wherein for the quantification
    a) the amount of antibodies, which is in excess relative to the sample, is reacted with an excess of labeled antigens, in addition to the antigens present in the sample to be investigated, and
    b) the resulting product is separated into antigenantibody complex and supernatant, and
    c) the amount of labeled antigen is determined either in the complex or in the supernatant after removal of the complex.

6. The method as claimed in claim 5, wherein the labeled antigen is radioactively, enzyme- or fluorescence-labeled.

7. The method as claimed in claim 4, wherein for the quantification
    a) the amount, which is in excess relative to the sample, of antibody, is reacted with an excess of antigen which is bound to a carrier, and then
    b) the antigen-antibody complex from a) is reacted with an excess of a second, labeled, antibody, and
    c) the product resulting from b) is separated into antigen-antibody complex and supernatant containing free, labeled, second antibodies, and

d) the amount of the second, labeled, antibody is determined either in the complex or in the supernatant.

8. The method as claimed in claim 8, wherein the second, labeled, antibody is radioactively, enzyme- or fluorescent-labeled.

**Revendications**

**Revendications pour les Etats contractants suivants : BE CH DE FR GB IT LI LU NL SE**

1. Procédé pour la préparation de protéoglycane héparanesulfate de basse densité ($\rho$ = 1,33-1,39), caractérisé en ce que

   a) on homogénéise et extrait un tissu à membranes basales, provenant de placenta humain, avec une solution d'un sel alcalin 3,4-4 M et/ou avec un solution d'un sel alcalin 0,1-1 M en présence d'inhibiteurs de protéases,

   b) on extrait le résidu avec une solution de sel chaotrope 1-8 M, en présence d'inhibiteurs de protéases, et à partir de l'extrait obtenu,

   c) on purifie ledit protéoglycane héparane-sulfate de basse densité, par chromatographie d'échange d'ions avec chromatographie subséquente sur tamis moléculaire et séparation en gradient de densité.

2. Procédé pour la production de sérums hautement spécifiques comportant des anticorps dirigés contre le protéoglycane héparane-sulfate de basse densité ($\rho$ = 1,33-1,39) préparable selon la revendication 1, caractérisé en ce que l'on immunise des animaux expérimentaux avec ledit protéoglycane héparane-sulfate de basse densité et on recueille leur sérum.

3. Procédé pour le dosage immunologique de protéoglycane héparane-sulfate de basse densité, caractérisé en ce que l'on détermine l'antigène protéoglycane héparane-sulfate de basse densité et/ou ses déterminants antigéniques, dans des liquides corporels, à l'aide d'anticorps pouvant être obtenus selon la revendication 2.

4. Protéoglycane héparane-sulfate de basse den-sité ($\rho$ = 1,33-1,39), pouvant être obtenu selon le procédé de la revendication 1.

5. Sérum hautement spécifique comportant des anticorps dirigés contre le protéoglycane héparane-sulfate de basse densité ($\rho$ = 1,33-1,39), qui peut être obtenu selon le procédé de la revendication 2.

6. Procédé selon la revendication 3, caractérisé en ce que

   a) on met en réaction ledit antigène ou ses déterminants antigéniques dans des liquides corporels, avec une quantité déterminée d'anticorps, en excès par rapport à l'échantillon à étudier, avec formation d'un complexe antigène-anticorps, et

   b) on détermine la quantité desdits anticorps à partir dudit complexe antigène-anticorps.

7. Procédé selon la revendication 6, caractérisé en ce que, pour la détermination quantitative,

   a) on met en réaction la quantité d'anticorps, en excès par rapport à l'échantillon, avec un excès d'antigènes marqués, en plus des antigènes présents dans l'échantillon à étudier, et

   b) on scinde le produit résultant en complexe antigène-anticorps et surnageant, et

   c) on détermine la quantité de l'antigène marqué, soit dans le complexe, soit dans le surnageant, après séparation du complexe.

8. Procédé selon la revendication 3, caractérisé en ce que l'antigène marqué est marqué par marquage radioactif, enzymatique ou fluorescent.

9. Procédé selon la revendication 6, caractérisé en ce que, pour la détermination quantitative,

   a) on met en réaction la quantité d'un anticorps, en excès par rapport à l'échantillon, avec un excès d'antigène qui est fixé sur un support, et ensuite

   b) on met en réaction le complexe antigène-anticorps provenant de a) avec un excès d'un second anticorps marqué, et

   c) on scinde le produit résultant de b) en complexe antigène-anticorps et surnageant à seconds anticorps marqués libres, et

   d) on détermine la quantité du second anticorps marqué, soit dans le complexe, soit dans le surnageant.

10. Procédé selon la revendication 8, caractérisé en ce que le second anticorps marqué est marqué par marquage radioactif, enzymatique ou fluorescent.

**Revendications pour l'Etat contractant suivant: AT**

1. Procédé pour la préparation de protéoglycane héparane-sulfate de basse densité ($\rho$ = 1,33-1,39), caractérisé en ce que

   a) on homogénéise et extrait un tissu à membranes basales, provenant de placenta humain, avec une solution d'un sel alcalin 3,4-4 M et/ou avec une solution d'un sel alcalin 0,1-1 M en présence d'inhibiteurs de protéases,

   b) on extrait le résidu avec une solution de sel chaotrope 1-8 M, en présence d'inhibiteurs de protéases, et à partir de l'extrait obtenu,

   c) on purifie ledit protéoglycane héparane-sulfate de basse densité, par chromatographie d'échange d'ions avec chromatographie subséquente sur tamis moléculaire et séparation en gradient de densité.

2. Procédé pour la production de sérums hautement spécifiques comportant des anticorps dirigés contre le protéoglycane héparane-sulfate de basse densité ($\rho$ = 1,33-1,39) préparable selon la revendication 1, caractérisé en ce que l'on immunise des animaux expérimentaux avec ledit protéoglycane héparane-sulfate de basse densité et on recueille leur sérum.

3. Procédé pour le dosage immunologique de protéoglycane héparane-sulfate de basse densité, caractérisé en ce que l'on détermine l'antigène protéoglycane héparane-sulfate de basse densité et/ou ses déterminants antigéniques, dans des liquides corporels, à l'aide d'anticorps pouvant être obtenus selon la revendication 2.

4. Procédé selon la revendication 3, caractérisé en ce que

   a) on met en réaction ledit antigène ou ses déterminants antigéniques dans des liquides corporels, avec une quantité déterminée d'anticorps, en excès par rapport à l'échantillon à étudier, avec formation d'un complexe antigène-anticorps, et

   b) on détermine la quantité desdits anticorps à partir dudit complexe antigène-anticorps.

5. Procédé selon la revendication 4, caractérisé en ce que, pour la détermination quantitative,

   a) on met en réaction la quantité d'anticorps, en excès par rapport à l'échantillon, avec un excès d'antigènes marqués, en plus des antigènes présents dans l'échantillon à étudier, et

   b) on scinde le produit résultant en complexe antigène-anticorps et surnageant, et

   c) on détermine la quantité de l'antigène marqué, soit dans le complexe, soit dans le surnageant, après séparation du complexe.

6. Procédé selon la revendication 5, caractérisé en ce que l'antigène marqué est marqué par marquage radioactif, enzymatique ou fluorescent.

7. Procédé selon la revendication 4, caractérisé en ce que, pour la détermination quantitative,

   a) on met en réaction la quantité d'un anticorps, en excès par rapport à l'échantillon, avec un excès d'antigène qui est fixé sur un support, et ensuite

   b) on met en réaction le complexe antigène-anticorps provenant de a) avec un excès d'un second anticorps marqué, et

   c) on scinde le produit résultant de b) en complexe antigène-anticorps et surnageant à seconds anticorps marqués libres, et

   d) on détermine la quantité du second anticorps marqué, soit dans le complexe, soit dans le surnageant.

8. Procédé selon la revendication 7, caractérisé en ce que le second anticorps marqué est marqué par marquage radioactif, enzymatique ou fluorescent.